# EUROPEAN PATENT SPECIFICATION

(11) **EP 0 552 255 B1**
(45) Date of publication and mention of the grant of the patent: **01.05.1996**
(21) Application number: 91918973.8
(22) Date of filing: 18.09.1991
(51) Int. Cl.: C07C 59/06, C07C 51/12, C07C 51/235, C07C 51/43

(54) **MANUFACTURE OF HIGH-PURITY HYDROXYACETIC ACID**
VERFAHREN ZUR HERSTELLUNG VON REINER HYDROXYESSIGSÄURE
FABRICATION D'ACIDE HYDROXYACETIQUE DE GRANDE PURETE

(30) Priority: 20.09.1990 US 585629
(43) Date of publication of application: 28.07.1993
(73) Proprietor: E.I. DU PONT DE NEMOURS AND COMPANY, Wilmington Delaware 19898 (US)
(72) Inventor: DiCOSIMO, Robert, Wilmington, DE 19808 (US)
(74) Representative: Jones, Alan John
(86) International application number: US9106626
(87) International publication number: WO9205138

(56) References cited:
- DE-A- 1 643 773
- DE-C- 2 911 944
- US-A- 2 037 654
- US-A- 2 152 852
- US-A- 2 153 064
- US-A- 3 859 349
- Römpps Chemie-Lexikon, Acthe, neubearbeitete und erweiterte Auflage, pages 22444-2245, Otto-Albrecht Neumüller: "Kristallisation".

## Description

The invention relates to a process for the manufacture of high-purity crystalline, hydroxyacetic acid from crude hydroxyacetic acid.

Hydroxyacetic acid (HAA) is a useful commercial acid which is typically used for many industrial applications, such as metal cleaning, metal complexing and electroplating. For these applications a technical grade hydroxyacetic acid is sufficient. However, in applications where hydroxyacetic acid is used in chemical synthesis, a purity of greater than 99% is highly desirable. An increased need for such high-purity HAA has been seen in recent years.

DE-A-1643773 discloses a process for preparing substantially pure glycolide from recrystallized partially purified glycolide containing some impurities. A layer of the partially purified glycolide is heated at a temperature of from 75°C to 130°C, under a pressure not greater than 2.5 mm of mercury, absolute, while providing a cooling surface positioned vertically immediately above and separated from the partially purified glycolide at a distance of no greater than 25 cm. The cooling surface is cooled constantly to a temperature not greater than 42°C, whereby substantially pure glycolide is sublimed from the partially purified glycolide and becomes deposited in substantially pure form on the cooling surface. The substantially pure glycolide is recovered from said cooling surface.

The present invention is an improvement in the existing and practised process for the manufacture of HAA by the carbonylation of formaldehyde and water in HAA with a sulfuric acid catalyst, where a crystallization step is inserted into the purification process described in US-A-3,859,349 after the anion exchange step, or after the stripper, or after the cation exchange step. With the insertion of the crystallisation step, high-purity crystalline HAA is produced. By high-purity is meant a purity sufficient to be used in chemical synthesis. This usually means a purity of 99% or greater.

According to this invention a process for the manufacture of crystalline hydroxyacetic acid (HAA) of greater than 99.3% purity utilising as the starting material 70% technical grade HAA with about 62.4 wt.% HAA, 8.8 wt.% HAA dimer, 2.2 wt.% diglycolic acid, 2.2 wt.% methoxyacetic acid, and 0.24 wt.% formic acid comprises cooling said technical grade HAA to a temperature from 10°C to -25°C, adding sufficient high-purity, crystalline HAA to the cooled solution to induce crystallization, and recovering the crystalline HAA.

The amount of pure HAA added can be as small as 0.01% by weight of the HAA present in the cooled or supercooled crude HAA solution.

The present invention is an improvement over other methods because it affords HAA in greater than 99.3% purity, is simple to perform, is inexpensive to operate, does not require the use of hazardous organic solvents, is readily adaptable to large scale operations for use in a batch or continuous fashion and provides pure HAA as a dry solid which can be more conveniently used than aqueous solutions.

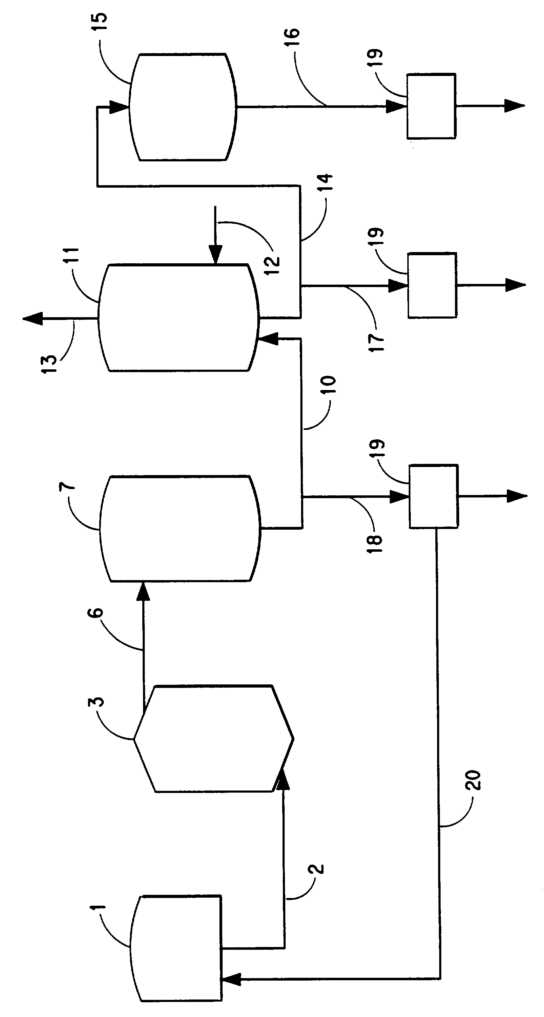

### DESCRIPTION OF THE DRAWING

The drawing is a flow chart of the process of this invention.

### DETAILED DESCRIPTION OF INVENTION

The process of this invention starts with the carbonylation of formaldehyde in water, in the presence of an organic acid, usually HAA, with sulfuric acid as a catalyst, and at pressures between 6,000 to 10,000 psig and temperatures of 210°C to 240°C. This carbonylation process is well known and is disclosed in U.S. Patents 2,153,064; 2,152,852 and 2,037,654. This process produces a crude hydroxyacetic acid which must be purified prior to use or sale.

The crude hydroxyacetic acid made by the above carbonylation process will have the following typical composition in weight percent:

| | |
|---|---|
| hydroxyacetic acid | 85% |
| formic acid | 1.5 |
| sulfuric acid | 1.5 |
| formaldehyde | 1.5 |
| water | 10.0 |
| methanol | 0.5 |

The crude hydroxyacetic acid produced by this carbonylation process is purified by the process disclosed in U.S. Patent 3,859,349. In this process the crude hydroxyacetic acid is first contacted with granulated activated carbon in a pulsed or fixed bed for color removal, the sulfuric acid present is then removed using a weak anion exchange column, low-boiling impurities (i.e., formaldehyde, formic acid, and methanol) are removed by heating at 110°C to 120°C, and finally, a cation exchange resin is used to remove trace metals. Further description of this process can be found in U.S. Patent 3,859,349.

The final product is a 70% technical-grade aqueous solution; a typical analysis of this material is:

| | |
|---|---|
| total acid as HAA (wt. %) | 71.3% |
| free acid as HAA (wt. %) | 62.4% |
| formic acid (wt. %) | 0.24% |
| color (Gardner) | 3 |
| chloride (ppm) | 2.6 |
| iron (ppm) | 0.2 |

Hydroxyacetic acid is nonvolatile and cannot be distilled even under reduced pressure. Heating molten HAA readily produces polyhydroxyacetic acid (polyglycolide) and water via a self-esterification reaction, thus distillation as a method of purification of HAA is not possible. Aqueous solutions of HAA are made up of mixtures of monomeric HAA and soluble polyacids (predominantly hydroxyacetic acid dimer) in equilibrium, the ratio being determined by solution concentration. The polyacids can be hydrolyzed upon dilution of 70% HAA with water to 20% by weight or less, and refluxing or the addition of alkali to this diluted solution markedly increases the rate of this hydrolysis. Although this dilution procedure can be used to convert the HAA dimer to HAA monomer, a number of other impurities are also present in the 70% technical grade HAA; the components of a typical sample of 70% HAA are listed below:

| | |
|---|---|
| HAA (wt. %) | 62.4 |
| HAA dimer (wt. %) | 8.8 |
| diglycolic acid (wt. %) | 2.2 |
| methoxyacetic acid (wt. %) | 2.2 |
| formic acid (wt. %) | 0.24 |

The desired high-purity HAA is obtained by employing a crystallization procedure after the anion exchange step, after the stripper step, or preferably, after the cation exchange step.

In the crystallization procedure, the crude HAA solution is cooled to a temperature of from 10°C (the reported melting point of 70% HAA) to -25°C (the temperature at which a supercooled solution of 70% HAA begins to solidify or freeze), or more preferably to a temperature between 5°C and -18°C (the latter temperature being that at which the viscosity of the solution is observed to increase considerably). To the cooled solution is then added sufficient pure, crystalline HAA to seed the solution and induce crystallization; the amount of pure HAA added can be as small as 0.01% by weight of the HAA present in the cooled or supercooled crude HAA solution.

Yields of high-purity HAA of from 6.6% to 24% are obtained by this method and the purity of the HAA obtained ranges from 99.3% to greater than 99.9%, depending on the reaction conditions. Although it is known that solutions of HAA in water at relatively high concentration may supercool below the freezing point of the solution, it was unexpected that HAA of such high purity could be crystallized by the seeding of a cooled or supercooled solution which contains such high levels of undesirable impurities (i.e., diglycolic acid, methoxyacetic acid, formic acid, and glycolic acid dimer). In the absence of added pure, crystalline HAA to seed the cooled or supercooled solution, crystallization of HAA may not readily occur; 70% technical grade HAA has been stored at 5°C for many weeks without crystallization of HAA.

The rate at which crystallization occurs, and the size of the resulting crystals, can be partially controlled by selecting the appropriate temperature, and by either agitating or not agitating the seeded, cooled solution. Crystallization is slower and yields of crystalline HAA are lower at higher temperatures (i.e., 5°C as opposed to -18°C); however, the highest-purity crystalline HAA is attained under these conditions. Agitation of the solution at a desired temperature will result in faster crystal formation, but the size of the crystals will be much smaller than those obtained without agitation. The crystal size is important when considering filtration requirements; if the crystals are to be filtered and not washed to remove the mother liquor (which contains the undesirable impurities), a large crystal size will hold up less of this mother liquor. It is advantageous to produce pure HAA without a wash to remove the mother liquor from the crystals. The highest purity crystalline HAA is obtained at higher temperatures (e.g., 5°C) and no stirring, although the yields are much less than those obtained at lower temperatures, with or without agitation. If it is desirable to remove the mother liquor from the crystals by washing, cold water (2°C to 5°C), cold acetone (< 5°C), or ethyl ether can be used as a washing solvent, although other washing solvents may work equally as well. The process of this invention could be used in a batch or continuous fashion.

The mother liquor recovered from the first crystallization can be recycled to the carbonylation reactor in the HAA manufacturing process, as a source of the required organic acid (see above), or a second crop of crystalline HAA may be obtained from this solution by first concentrating the mother liquor, then repeating the cooling and seeding procedure (an example of such a procedure appears below). Evaporation of the mother liquor may be performed at atmospheric pressure or under reduced pressure.

The process of the invention will now be described with reference to the figure.

Crude hydroxyacetic acid is made by the carbonylation process in reactor 1. The crude acid flows from through conduit 2 to absorber 3 which is packed with activated granular carbon. Decolorized crude acid 6 leaves the top of the absorber and is fed into the top of an anion exchange column 7 wherein the sulfuric acid present is removed. This column employs a weak anion exchange resin.

The effluent 10 from the anion exchanger 7 is fed into a stripper 11. Live steam 12 is sparged into the stripper and low boiling impurities are taken off overhead 13. The bottoms of the stripper are fed to a cation exchange column 15 wherein the resin removes trace metals.

The technical grade acid 16 from the cation exchanger is fed into a crystallizer 19 where the solution is cooled and, solid HAA of high purity is recovered.

Alternatively effluent 18 from the anion exchanger 7 or bottoms 17 from stripper 11 can be fed to a crystallizer 19. Mother liquor from the crystallizer can be recycled to the reactor 1 as illustrated by line 20.

### EXAMPLES

### Example 1

Into a 15 mL polypropylene centrifuge tube was placed 4.0 mL of 70% HAA (3.89 g hydroxyacetic acid). The tube and its contents were cooled to 5°C, then a few crystals of hydroxyacetic acid (Aldrich, 99% pure; < 2-3 mg) were added to the tube and the solution maintained at 5°C for 24 h. During this time a number of large (3-4 mm) hexagonal crystals were produced, and were isolated by filtering the solution (no washing was performed) to yield 0.255 g (6.6% isolated yield) of hydroxyacetic acid. Analysis of this crystallized HAA by HPLC indicated it was greater than 99.9% pure hydroxyacetic acid; no formate, methoxyacetate, or diglycolic acid were detected.

### Example 2

Into a 100 mL Erlenmeyer flask was placed 50 mL of 70% HAA (43.2 g of hydroxyacetic acid), then the flask and its contents were cooled to -10 to -15°C in a dry ice/acetone bath. To the resulting viscous solution was added hydroxyacetic acid (Aldrich, 99% pure; ca. 20 mg) and the solution was mixed by swirling the flask while maintaining the temperature at -10 to -15°C in the dry ice/acetone bath. Within a few minutes a fine white precipitate was observed, and after ca. 15 minutes of mixing at -10 to -15°C, the cold solution was vacuum filtered, and the white solid allowed to air dry on the filter paper (it was not washed). The resulting white powder (10.2 g, 23.6% isolated yield) was analyzed by HPLC: hydroxyacetic acid, 99.4%; diglycolic acid, 0.4%; methoxyacetic acid, 0.2%; dimer, 0%.

### Example 3

Into a 1000-mL Erlenmeyer flask was placed 500 mL of 70% HAA (432 g of hydroxyacetic acid). The flask and its contents were cooled to 5°C, then hydroxyacetic acid (Aldrich, 99% pure; ca. 50 mg) was added and the solution allowed to stand at 5°C for 18 h. Large hexagonal crystals were observed to have formed, but most likely not in any greater yield than the 6.6% yield observed earlier at 5°C. The flask was cooled to -18°C over a period of 1 h, then the contents of the flask were mixed by vigorously swirling the flask intermittently over the next 5 h while maintaining the solution at -18°C. A fine, white crystalline solid was observed to precipitate during this time, and was collected by vacuum filtration and washed with ca. 100 mL of ethyl ether. The solid was dried under vacuum, then weighed (71.9 g, 16.7% yield), and subsequently analyzed by HPLC: hydroxyacetic acid, 99.3%; diglycolic acid, 0.6%; methoxyacetic acid, 0.1%; dimer, 0%.

### Example 4

A 1-quart plastic bottle of 70% Hydroxyacetic acid (technical grade) from Belle (containing 1176 grams of solution) was placed in a freezer at -18°C for 4 hours, then 0.100 g of recrystallized HAA (99.3% pure) was added. The contents of the bottle were gently mixed to disperse the added HAA, then the bottle was stored in a freezer at -18°C for 63 h without agitation of the mixture. The resulting crystalline solid was collected by vacuum filtration of the mixture on a course-porosity glass fritted funnel, and the crystals allowed to air dry on the funnel (they were not washed). The crystals were then dried to a constant weight under vacuum at room temperature to yield 225 g of hydroxyacetic acid (27% isolated yield), which was analyzed by HPLC: hydroxyacetic acid, 99.9%; diglycolic acid, 0.1%; methoxyacetic acid, 0%; dimer, 0%.

A second crop of crystalline HAA was obtained from the remaining mother liquor (filtrate) from the first crystallization by first reducing the volume of this filtrate from 785 mL to 690 mL by boiling down the solution. This solution was then cooled to -18°C for 4 h, then 0.10 g of recrystallized HAA was added to the solution and the mixture maintained at -18°C for an additional 22 h. The resulting crystals were collected by vacuum filtration (143 g, 17% isolated yield). Analysis of this second crop by HPLC gave the following results: HAA, 98.2%; HAA dimer, 1.2%; diglycolic acid, 0.3%; methoxyacetic acid, 0.3%. The HAA dimer was hydrolyzed to HAA by adjusting the pH of a solution of this 2nd crop to > 10 with conc. sodium hydroxide: HAA, 99.3%; HAA dimer, 0%; diglycolic acid, 0.3%; methoxyacetic acid, 0.4%.

### Example 5

Into a 100 mL erlenmeyer flask was placed 50 mL of 70% HAA (43.2 g of hydroxyacetic acid), then the flask and its contents were cooled to -9°C. To the resulting viscous solution was added hydroxyacetic acid (Aldrich, 99% pure; ca. 20 mg) and the resulting mixture was maintained at -9°C without stirring or agitation. After 18 h the mixture was vacuum filtered using a fritted-glass funnel (the solid was not washed), and the crystalline solid dried under vacuum (1-2 mm Hg) to a constant weight at 26°C. The resulting white crystalline solid (5.54 g, 12.8% isolated yield) was analyzed by HPLC: hydroxyacetic acid, 99.7%; diglycolic acid, 0.3%; methoxyacetic acid, 0%; dimer, 0%.

The crystallization described above was repeated, except that the seeded solution was maintained at -9°C for 65 h before filtering. The resulting white crystalline solid (8.22 g, 19.0% isolated yield) was analyzed by HPLC: hydroxyacetic acid, 99.8%; diglycolic acid, 0.2%; methoxyacetic acid, 0%; dimer, 0%.

## Claims

1. A process for the manufacture of crystalline hydroxyacetic acid (HAA) of greater than 99.3% purity utilizing as the starting material 70% technical grade HAA with about 62.4 wt.% HAA, 8.8 wt.% HAA dimer, 2.2 wt.% diglycolic acid, 2.2 wt.% methoxyacetic acid, and 0.24 wt.% formic acid comprising:
(i) cooling said technical grade HAA to a temperature from 10°C to -25°C,
(ii) adding sufficient high-purity, crystalline HAA to the cooled solution to induce crystallization, and
(iii) recovering the crystalline HAA.

2. The process of claim 1 wherein the 70% technical grade HAA is cooled from 5°C to -18°C.

3. The process of claim 1 or claim 2 wherein the crystalline HAA is recovered by filtration.

## Patentansprüche

1. Verfahren zur Herstellung kristalliner Hydroxyessigsäure (HAA) mit einer Reinheit von über 99,3 %, das als Ausgangsmaterial 70 % technisch reine HAA mit etwa 62,4 Gew.-% HAA, 8,8 Gew.-% HAA-Dimer, 2,2 Gew.-% Diglycolsäure, 2,2 Gew.-% Methoxyessigsäure und 0,24 Gew.-% Ameisensäure verwendet und umfaßt:
(i) Abkühlen der technisch reinen HAA auf eine Temperatur von 10 °C bis -25 °C,
(ii) Zugeben von genügend hochreiner kristalliner HAA zu der abgekühlten Lösung, um die Kristallisation auszulösen, und
(iii) Gewinnen der kristallinen HAA.

2. Verfahren nach Anspruch 1, bei dem die 70%ige technische HAA von 5 °C auf -18 °C abgekühlt wird.

3. Verfahren nach Anspruch 1 oder Anspruch 2, bei dem die kristalline HAA durch Filtration gewonnen wird.

## Revendications

1. Procédé de fabrication d'acide hydroxyacétique cristallin (AHA) de pureté supérieure à 99,3 % utilisant comme matériau de départ un AHA de qualité technique à 70% avec environ 62,4 % en poids de AHA, 8,8 % de dimère de AHA, 2,2 % en poids d'acide diglycolique, 2,2 % en poids d'acide méthoxyacétique, et 0,24 % en poids d'acide formique comprenant les étapes de :
(i) refroidissement dudit AHA de qualité technique à une température de 10°C à -25°C,
(ii) addition suffisante de AHA cristallin, de pureté élevée à la solution refroidie pour provoquer la cristallisation, et
(iii) récupération du AHA cristallin.

2. Procédé selon la revendication 1 dans lequel le AHA de qualité technique à 70 % est refroidi de 5°C à -18°C.

3. Procédé selon la revendication 1 ou la revendication 2 dans lequel le AHA cristallin est récupéré par filtration.
